# EUROPEAN PATENT APPLICATION

(11) **EP 2 016 966 A1**
(43) Date of publication of application: **21.01.2009**
(21) Application number: 07013892.0
(22) Date of filing: 16.07.2007
(51) Int. Cl.: A61M 16/00

(54) **Patient-ventilator synchronization apparatus**

(71) Applicant: MAP Medizin-Technologie GmbH, 82144 Martinsried (DE)
(72) Inventor: Burz, Johann S., 82407 Wielenbach/Wilzhofen (DE); Biener, Achim, 85445 Aufkirchen (DE); Lang, Bernd, 82166 Gräfelfing (DE)
(74) Representative: Vossius & Partner

(57) **Abstract**

The invention relates to a method and apparatus for supply and preferably for therapeutic supply of breathable air to a patient. More specifically, the invention relates to a method and apparatus for synchronizing patient respiration with a ventilator (3) so that the phase of the respiratory cycle of the patient can match the respiratory support of a ventilator (3). In particular, there is provided an apparatus (1) for supply of breathable gas to a patient, wherein the apparatus comprises detection means (2) for detecting at least one parameter of the patient, ventilation means (3) for providing breathable air depending on the detected parameter of the patient, and synchronizing means (4) for synchronizing patient and device wherein the synchronizing means (4) submits a synchronizing signal to the patient.

## Description

The invention relates to a method and apparatus for supply and preferably for therapeutic supply of breathable air to a patient. More specifically, the invention relates to a method and apparatus for synchronizing patient respiration with a ventilator so that the phase of the respiratory cycle of the patient can match the respiratory support of a ventilator.

Methods for supporting a patient's ventilation can be classified in invasive ventilation and non-invasive ventilation using either positive or negative pressure.

In the art, different approaches for supporting a patient's ventilation are known, wherein the phases of breath support or assistance may be based, e.g., on the volume of exhaled or inspirited air, on the pressure of expiration or inspiration, or on the oxygen saturation.

The approaches and therapies as referred to above and as well know in the art still suffer from disadvantages. In particular, a lack of synchrony between the respiratory cycle of the patient and that of the ventilator used to assist the patient by providing air pressure and/or flow pattern (in a certain frequency) can lead to patient discomfort. E.g. in cases in which non-invasive pressure and/or flow pattern ventilation is used as an alternative to invasive positive pressure respiration, the patient often has unlearned spontaneous breathing and needs to get used to controlled, e.g., non-invasive, ventilation, e.g., via a respiration mask. For example, non-invasive positive pressure ventilation is used as an alternative to invasive positive pressure respiration via, e.g., an endotracheal intubation. In this case the object of a therapeutic ventilation is, e.g., wean off the patient from the use of an invasive supply of breathing gas.

Many attempts have been made to improve devices for providing pressure and/or flow pattern treatment by sensing various parameters of the patient and/or the patient's breath and by adapting the provided air pressure and/or air flow accordingly.

For example, ventilators assisting spontaneously breathing patients strive to synchronize their performance with the patient's efforts. To do this, ventilators typically measure one or more of pressure, volume, flow and time and compare that measure with predetermined thresholds. The ventilator may then adjust the pressure, volume or flow of air being delivered to the patient in accordance with the patient's efforts. For example, a flow-triggered pressure controlled device may deliver air at one fixed pressure to a patient until the flow crosses a threshold level, whereupon the pressure is changed to another fixed pressure. Depending on their conditions, different patients may experience different levels of discomfort depending upon how quickly and accurately the ventilator tracks the patients' efforts.

Thus, there still exists the need to improve the existing systems. Accordingly, it is an object underlying the present invention to provide a method and an apparatus that improves the patient's comfort in the field of the provision of breathable air to a patient, e.g. via non-invasive positive pressure ventilation, that overcome the disadvantages of the prior art. Moreover or additionally it is an object underlying the present invention to provide an apparatus and a method that assist patients in weaning of the effects of controlled or assisted breathing. This particularly to allow the patient to comfortably change from, e.g., controlled breathing to assisted breathing or to free or spontaneous breathing or from assisted breathing to free or spontaneous breathing.

The object underlying the present invention is achieved by the features of the independent claims. The dependent claims relate to preferred embodiments of the present invention.

The invention relates to a method and apparatus for delivering a supply of breathable air at, e.g., positive pressure to a patient wherein the apparatus comprises detection means for detecting at least one parameter of the patient, ventilation means for providing breathable air depending on the detected parameter of the patient, and synchronizing means for synchronizing patient and device wherein the synchronizing means submits information, e.g. in the form of a synchronizing signal, from the device to the patient. Preferably, the submitted information regards the provision of breathable air to the patient and more preferably, the submitted information regards the incipient provision of breathable gas or a change in the respiration phase of the ventilation.

The patient parameters are indicative of the patient's breathing and the need for respiration such as the patient's efforts to breath, the patient's supply with oxygen etc. Such parameters as well as means for sensing such parameters are well known in the art.

According to a preferred embodiment, the apparatus further comprises a breath signal channel, on which the parameters of the patient and/or the breathable air are submitted and/or exchanged as well as a synchronizing channel on which the transfer of the information or synchronizing signal takes place wherein the breath signal channel and the synchronizing channel are separated, distinct and/or independent from each other.

Preferably, the information or signal submitted by the synchronizing means anticipates a phase change in the respiration cycle of the ventilation means. The synchronizing signal is preferably suitable to inform a patient about the respiration cycle of the ventilation, e.g. the start or end of an inspiration phase or an expiration phase and/or to stimulate or motivate the patients spontaneous breathing. Accordingly, the information or signal submitted by the synchronizing means indicates that the ventilation means will change the phase and instructs or learns the patient to also change the phase of the respiration cycle.

The synchronizing signal is preferably submitted in visible, tangible and/or audible form. Preferably, the synchronizing information is submitted by means of light, audio, electric impulses, temperature, audio on the breathing gas stream and/or reflex stimulation.

More preferably, the synchronizing signal is directly and/or indirectly submitted to the patient. For example, the synchronizing information or signal reaches or triggers the patient's consciousness and/or sub-consciousness, the patient's reflexes, or the patient's brain. Thereby, e.g., a breathing reflex etc. of the patient may be directly stimulated or triggered. Additionally or alternatively, the synchronizing signal may be indirectly submitted to the patient. For example, the synchronizing signal may be independent from the patient's breathing system, such as a noise. Preferably, the synchronizing signal sets the rhythm or frequency of the breathing cycle. The respective device and method may also be advantageously used in the field of treating asthmatic patients or hyper-ventilating patients.

Preferably, independently of whether the signal is provided to the patient directly or indirectly, the synchronizing signal helps or teaches the patient to breath spontaneously, improves the patient's spontaneous breathing and/or synchronizes the patient's spontaneous breathing with the ventilation means so that the phase of the respiratory cycle of the patient can match the respiratory support of the ventilation means.

The apparatus, and method, according to the present invention preferably allows the application of two modes, which is a controlled ventilation mode as well as an assist mode. In the controlled ventilation mode the apparatus according to the present invention provides a constant ventilation or respiration of a patient and improves the synchronization of the respiration cycle of the patient and that of the apparatus. In the assist mode, the patient is, e.g., weaned off controlled breathing, such as during an invasive supply of breathing gas, and/or the device allows spontaneous breathing of the patient and/or the device teaches the patient to breath spontaneously. In the case of weaning off the effects of controlled and/or assisted breathing, e.g. during an intubation, the synchronizing signal anticipating and being independent from the breathing signal serves as a stimulant for effecting and synchronizing spontaneous breathing.

Preferably, the device is further adapted to recognize spontaneous breathing of the patient continuously or intermittently. Preferably, this is achieved by interrupting the breathing cycle, e.g., for at least one inspiration and/or expiration phase, and by checking whether the patient has spontaneous breathing based on the sensed patient parameters.

According to a further preferred embodiment, the present invention relates to a method for, e.g., non-invasively, supplying breathable gas to a patient, wherein at least one parameter of the patient is detected by detection means, breathable air is supplied to the patient depending on detected parameter of the patient by ventilation means and submitting information to the patient by a synchronizing means for synchronizing patient and device. Further embodiments of the preferred method correspond to those discussed with regard to the apparatus according to the present invention.

According to a further preferred embodiment, the present invention relates to a method for, e.g., non-invasively delivering a supply of air or breathable gas at positive pressure to a patient comprising the steps of detecting at least one parameter of a patient, supplying breathing gas depending on the detected parameters of the patient and transmitting a synchronizing signal for synchronizing the device and the patient to the patient wherein the synchronizing signal comprises information relating the provision of breathing gas to the patient.

Further preferred embodiments and features of the inventive method correspond to the use of the device and to the features as discussed above with regard to the device according to the present invention.

The present invention is of particular advantage in that it provides an improved synchronization of patient and ventilation thereby improving the patients comfort and respiration, in that it supports and improves the patient's spontaneous breathing, and in that it achieves an effective wean off the effects of controlled and/or badly synchronized ventilation.

The method and device according to the present invention further advantageously allow adaptation of the parameters to the changing demands of the patient during weaning off.

In the following, the present invention will be discussed with reference to a preferred exemplary embodiment thereby referring to the figure which shows a schematic concept of a preferred embodiment of the device according to the present invention.

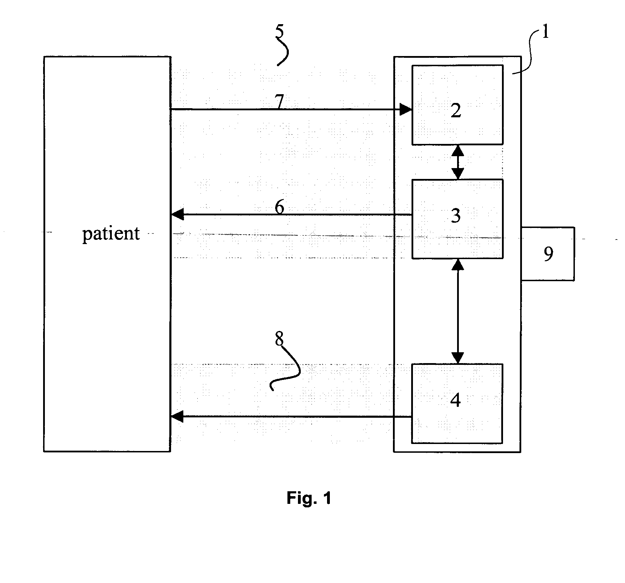

Figure 1 shows a device 1 for a non-invasive positive pressure ventilation of a patient comprising a detection means 2, a ventilation means 3 as well as a synchronizing means 4. The detection unit detects at least one parameter of the patient such as the oxygen level in the patient's blood, snore signal, and/or arterial oxyhaemoglobin saturation etc. indicative of a patients respiratory phase etc. The ventilation means 3 provides breathable air to the patient, thereby adjusting various parameters according to the patient's needs. Among these parameters are the oxygen content versus time in the patient's breath, breath pressure versus time and/or volume flow versus time. Breathable air is any fluid, gaseous and/or liquid, which is breathable by a patient.

The parameters of the patient as detected by the detecting means 2 as well as the breathing air and the corresponding parameters as referred to above are received from and submitted to the patient via a breath signal channel 5 in order to provide breathable air to patient so that the respiratory support of a ventilator can match the phase of the respiratory cycle of the patient. The breath signal channel preferably includes the air inhaled and exhaled by the patient, i.e., the pneumatic connection 6 between patient and apparatus as well as any further sensor lines 7 submitting parameters of the patient to the apparatus.

The synchronizing means 4 exchanges information with the detection 2 and/or ventilation 3 unit regarding detected parameters of the patient and the respiratory support of the ventilation means and particularly the respiratory cycle, particularly with regard to time, of the patient and/or the ventilation means.

Depending on the information received from the detection means 2, the ventilation means 3, the synchronizing means 4 provides a synchronizing signal to the patient via a synchronizing channel 8. The synchronizing signal comprises information regarding the next inspiration or expiration cycle as conducted by the ventilation means 3. The respective synchronizing signal preferably anticipates the breathing signal, i.e., it reaches the patient timely in advance of the breathing signal such as change in the respiration phase of the ventilation means 3. Preferably, the synchronizing signal comprises information regarding time or moment and/or duration of a breathing cycle or phase and/or the start or end of a breathing cycle or phase of the ventilation means 3. Furthermore the synchronizing signal is preferably determined on the basis of the detected parameters of the patient and/or based on the passing time if the apparatus 1 is run in a time-dependent or T-mode.

According to preferred embodiments of the present invention the synchronizing signal is provided to the patient by means of a signal, such as a light signal, audio signal, electric impulse signal, temperature signal etc. According to further preferred embodiments, the respiration mask may be active in that the signal is provided via the respiration mask, e.g., by changing the pressure in the mask's air cushions, either independent cushions provided for that purpose only or multifunctional air cushions also contributing, e.g., in supporting the mask on a patient's face, and thus applying a pressure on the patient's body etc. Preferably, an audio signal may be applied to the breathing gas stream or directly to the patients ear and/or breathing reflexes of the patient may be stimulated.

According to preferred embodiments of the present invention the synchronizing signal is provided to the patient via a synchronizing channel 8 which is separate from the breath signal channel 5. Preferably, the synchronizing signal is provided to the patient in a way that it reaches the patient's consciousness and/or sub-consciousness.

Accordingly, the synchronizing signal informs the patient, either directly or indirectly of a forthcoming respiration action, such as a phase change of the ventilation means 3 so that the patient can react accordingly. The patient can be educated or triggered to apply a certain respiration pattern thereby harmonizing and synchronizing the respiratory cycle of the patient to that of the ventilation device and thereby further improving the patient's comfort. Moreover, the provision of a synchronizing signal to the patient provides for weaning off the patient's addiction to the controlled or assisted breathing, e.g., the invasive intubation, thereby motivating and stimulating the patient to breath spontaneously.

Accordingly, by "warning" or "informing" the patient before the next expiration and/or inspiration tonus is applied by the device 1, the patient's constitution, convalescence and comfort is improved.

## Claims

1. Apparatus (1) for supply of breathable gas to a patient, wherein the apparatus comprises detection means (2) for detecting at least one parameter of the patient, ventilation means (3) for providing breathable air depending on the detected parameter of the patient, and synchronizing means (4) for synchronizing patient and device wherein the synchronizing means submits a synchronizing signal to the patient.

2. Apparatus according to claim 1, wherein the synchronizing means (4) submits information regarding the provision of breathable gas to the patient via the synchronizing signal.

3. Apparatus according to claim 1 or 2, further comprising a breath signal channel (5), on which the parameters of the patient and/or the breathable air are submitted and/or exchanged and a synchronizing channel (8) via which the information is transferred, wherein the breath signal channel (5) and the synchronizing channel (8) are separated from each other.

4. Apparatus according to any one of claims 1 to 3, wherein the synchronizing signal anticipates an action of the ventilation means (3).

5. Apparatus according to any one of claims 1 to 4, wherein the synchronizing signal is suitable to directly approach or address the patient.

6. Apparatus according to any one of claims 1 to 5, wherein the transmission and/or submission of the synchronizing signal is effected visually, tangible and/or audible.

7. Apparatus according to any one of claims 1 to 6, wherein the synchronizing information is transmitted and/or submitted as light-, audio-, electric impulses-, temperature-, audio on the breathing gas stream- and/or reflex stimulation-signal.

8. Apparatus according to any one of claims 1 to 7, wherein the transmission and/or submission may take place directly and/or indirectly.

9. Apparatus according to any one of claims 1 to 8, further comprising control means (9) for controlling detection means (2), ventilation means (3) and synchronizing means (4).

10. Apparatus according to any one of claims 1 to 9, wherein the synchronizing means (4) is connected to the detection means (2), the control means (9) and/or the ventilation means (3) in order to receive information regarding the next ventilation.

11. Apparatus according to any one of claims 1 to 10, wherein the synchronizing signal is transmitted and/or submitted independently from the breath signal channel (5).

12. Apparatus according to any one of claims 1 to 10, for non-invasive and/or invasive supply of breathable gas to a patient.

13. Method for non-invasively supplying breathable gas to a patient, wherein parameters of the patient are detected by detection means, breathable air is supplied to the patient depending on detected parameters of the patient by ventilation means and submitting information to the patient by a synchronizing means for synchronizing patient and device.
